# EUROPEAN PATENT APPLICATION

(11) **EP 4 098 271 A1**
(43) Date of publication of application: **07.12.2022**
(21) Application number: 21747952.6
(22) Date of filing: 27.01.2021
(51) Int. Cl.: A61K 35/74, A61P 35/00, A23L 33/135, A23K 10/16

(54) **PHARMACEUTICAL COMPOSITION FOR PREVENTING OR TREATING CANCER, COMPRISING WEISSELLA CIBARIA WIKIM28 AS ACTIVE INGREDIENT**

(30) Priority: 31.01.2020 KR 20200012144; 11.05.2020 KR 20200056138
(71) Applicant: Liscure Biosciences Co., Ltd., Seoul 06035 (KR)
(72) Inventor: CHIN, Hwa Sup, Yongin-si Gyeonggi-do 16824 (KR)
(74) Representative: Cabinet Becker et Associés
(86) International application number: PCT/KR2021/001059
(87) International publication number: WO 2021/153980

(57) **Abstract**

The present invention relates to a composition for preventing or treating cancer, comprising, as an active ingredient, *Weissella cibaria* WIKIM28 or a culture thereof isolated from kimchi.

The *Weissella cibaria* WIKIM28 or a culture thereof according to the present invention exhibits excellent anticancer activity, and thus can be usefully used as a composition for use in the treatment, prevention, or improvement of cancer in humans or animals.

## Description

### [Technical Field]

The present invention relates to a composition for prevention or treatment of cancer comprising *Weissella cibaria* WIKIM28 (Accession No. KCCM11879P) isolated from kimchi as an active ingredient.

### [Background Art]

Cancer shows a high mortality rate worldwide, and is the most common cause of death after cardiovascular disease in Western societies. In particular, due to westernization of dietary habits, the intake of high-fat diets has become common, and because of a rapid increase in environmental pollutants and an increase in alcohol consumption, and the like, colon cancer, breast cancer, prostate cancer and the like continue to increase, and lung cancer is increasing due to an increase of smoking population and air pollution in addition to aging of the population. In this situation, creation of anti-cancer substances which can contribute to enhancement of human health, improvement of healthy quality of life and improvement of human health, by enabling early prevention and treatment of cancer is urgently required.

Meanwhile, lactic acid bacteria are widely distributed in mouth, intestine, vagina and feces of humans and animals and fermented foods such as kimchi, and are closely related to the health of humans and animals. Lactic acid bacteria show various health promotion effects such as intestinal regulation, inhibition of harmful bacteria, immunoregulation, lowering of cholesterol in blood, anti-cancer activity, and the like.

So far, Korea Patent No. 10-2009742 discloses the anticancer activity of the *Weissella cibaria* strain, but only describes specific examples for the preventive or therapeutic effect on colorectal cancer, and specific experimental examples or embodiments are not described for other carcinomas.

### [Disclosure]

### [Technical Problem]

An object of the present invention is to provide a pharmaceutical composition for prevention or treatment of cancer comprising *Weissella cibaria* strain as an active ingredient.

In addition, other object of the present invention is to provide a food composition or food additive composition for prevention or improvement of cancer comprising a novel kimchi lactic acid bacterium, *Weissella cibaria* strain as an active ingredient.

Furthermore, other object of the present invention is to provide a feed composition or feed additive composition for prevention or improvement of cancer comprising a novel kimchi lactic acid bacterium, *Weissella cibaria* strain as an active ingredient.

### [Technical Solution]

Accordingly, the present inventors received the *Weissella cibaria* WIKIM28 strain (accession number: KCCM11879P) deposited at the Korean Culture Center of Microorganisms and tried to confirm the preventive and therapeutic effects on carcinomas other than colorectal cancer. As a result, the present invention was completed by confirming the preventive and therapeutic effects on other carcinomas.

In order to achieve the objects, the present invention provides a pharmaceutical composition for prevention or treatment of cancer comprising *Weissella cibaria* WIKIM28 (accession number KCCM11879P) or its culture as an active ingredient.

Furthermore, the present invention provides a food composition or food additive composition for prevention of improvement of cancer comprising *Weissella cibaria* WIKIM28 (accession number KCCM11879P) or its culture as an active ingredient.

Moreover, the present invention provides a feed composition or feed additive composition for prevention or improvement of cancer of livestock comprising *Weissella cibaria* WIKIM28 (accession number KCCM11879P) or its culture as an active ingredient.

### [Advantageous Effects]

*Weissella cibaria* WIKIM28 according to the present invention shows excellent anti-cancer activity against various carcinomas, and therefore it can be usefully used as a composition in use of treatment, prevention or improvement of cancer of humans or animals.

### [Description of Drawings]

FIG. 1 is a diagram showing a graph measuring cell growth rate (cell viability assay) after non-small lung cancer cells H1650 and A549 cell lines were treated with a composition according to the present invention.
FIG. 2 is a diagram showing a graph measuring cell growth rate (cell viability assay) after a pancreatic cancer AsPC-1 cell line was treated with a composition according to the present invention.
FIG. 3 is a diagram showing a graph measuring cell growth rate (cell viability assay) after a bladder cancer T24 cell line was treated with a composition according to the present invention.
FIG. 4 is a diagram showing a graph measuring cell growth rate (cell viability assay) after melanoma B16-F10, A375P, and A375SM cell lines were treated with a composition according to the present invention.
FIG. 5 is a diagram showing a graph measuring cell growth rate (cell viability assay) after a normal NIH/3T3 cell line was treated with *Weissella cibaria* WIKIM28 live cells.

### [Mode for Invention]

Hereinafter, the present invention will be described in more detail by examples. These examples are only intended to illustrate the present invention more specifically, and it will be obvious to those skilled in the art that the scope of the present invention is not limited by these samples according to the gist of the present invention.

The composition including *Weissella cibaria* WIKIM28 (Weissella cibaria, WIKIM28, accession number KCCM11879P) of the present invention or a culture thereof as an active ingredient has a preventive or therapeutic effect on cancer, and can be used as a pharmaceutical composition.

*Weissella cibaria* WIKIM28 of the present invention is a lactic acid bacteria strain derived from kimchi. The *Weissella cibaria* WIKIM28 is a probiotic and has a general intestinal effect and immune enhancing effect of lactic acid bacteria. It is a well-known fact that the lactic acid bacteria of the genus Lactobacillus have an effect of enhancing the intestinal effect and immune-enhancing effect.

The *Weissella cibaria* WIKIM28 is characterized in that it has a nucleic acid sequence represented by SEQ ID NO: 1.

The *Weissella cibaria* WIKIM28 can be used by inoculating in 0.1 to 10% in MRS liquid medium and culturing at 25°C to 37°C for 4 hours to 48 hours.

The culture method is preferably a stationary culture method, but is not limited thereto.

Herein, 'probiotics' are understood to mean living microorganisms that have a beneficial effect on health of the host by improving the host's intestinal microbial environment in the gastrointestinal tract of animals including humans. Probiotics are living microorganisms with probiotic activity and are in the form of single or complex strains, and when fed to humans or animals in the form of dried cells or fermented products to humans or animals, they can have a beneficial effect on the intestinal flora of the host.

The cancer may be any one selected from the group consisting of bladder cancer, breast cancer, melanoma, thyroid cancer, parathyroid cancer, rectal cancer, throat cancer, laryngeal cancer, esophageal cancer, pancreatic cancer, stomach cancer, tongue cancer, skin cancer, brain tumor, uterine cancer, gallbladder cancer, oral cancer, colon cancer, anal region cancer, liver cancer, lung cancer and colorectal cancer, and preferably, it may be colorectal cancer, but not limited thereto.

The *Weissella cibaria* WIKIM28 comprised in the composition according to the present invention may be present as a live cell or dead cell, and in addition, it may be present in a dried or freeze-dried form. Forms and formulation methods of lactic acid bacteria suitable for inclusion in various compositions are well known to those skilled in the art.

The composition may be administered orally or parenterally. In case of parenteral administration, it may be administered by intravenous injection, subcutaneous injection, intramuscular injection, intraperitoneal injection, endothelial administration, local administration, intranasal administration and rectal administration, and the like, and preferably, it may be administered by intravenous injection, but not limited thereto.

The appropriate dose of the present invention may be prescribed in various ways depending on factors such as formulation method, patient's age, body weight, gender, pathological condition, food, administration time, administration route, excretion rate and response sensitivity.

When the composition of the present invention is utilized as a pharmaceutical composition, the pharmaceutical composition of the present invention may be prepared by using a pharmaceutically appropriate and physiologically acceptable adjuvant in addition to the active ingredient, and as the adjuvant, an excipient, disintegrating agent, sweetener, binding agent, coating material, expansion agent, lubricant, glidant or flavoring agent, or the like may be used.

The pharmaceutical composition may be preferably formulated as a pharmaceutical composition by additionally comprising one or more kinds of pharmaceutically acceptable carriers in addition to the described active ingredient for administration.

For example, for formulation in a form of a tablet or capsule, the active ingredient may be bound to an oral and non-toxic, pharmaceutically acceptable inactive carrier, such as ethanol, glycerol, water and the like. In addition, when desired or needed, a suitable binding agent, a lubricant, a disintegrating agent and a coloring agent may also be comprised in the mixture. The suitable binding agent is not limited thereto, but includes starch, gelatin, natural sugars such as glucose or beta-lactose, natural and synthetic gum such as corn sweetener, acacia, tragacanth or sodium oleate, sodium stearate, magnesium stearate, sodium benzoate, sodium acetate, sodium chloride, and the like. The disintegrating agent is not limited thereto, but includes starch, methyl cellulose, agar, bentonite, xanthan gum, and the like. As the pharmaceutically acceptable carrier in the composition to be formulated as a liquid solution, saline solution, sterile water, Ringer's solution, buffered saline solution, albumin injection solution, dextrose solution, maltodextrin solution, glycerol, ethanol and a mixture of one or more components among them may be used, and if necessary, other common additive such as an anti-oxidant, buffer solution, a bacteriostatic agent, and the like may be added. In addition, it may be formulated as a formulation for injection such as aqueous solution, suspension, emulsion, etc., pill, capsule, granule or tablet, by additionally adding a diluent, a dispersing agent, a surfactant, a binding agent and a lubricant.

Furthermore, as an appropriate method in the field, using the method disclosed in Remington's Pharmaceutical Science, Mack Publishing Company, Easton PA, it may be preferably formulated depending on each disease or component.

The composition comprising *Weissella cibaria* WIKIM28 (Accession number KCCM11879P) or its culture as an active ingredient of the present invention may be used as a food composition or food additive composition for prevention or improvement of cancer.

The food composition may be a form of health functional food. The "health functional food" means a food produced and processed using raw materials or ingredient having useful functions to human bodies in accordance with the Health Functional Food Act (Article 3, No. 1), and the "functionality" means to obtain useful effects for health uses such as regulating nutrients or physiological effects, and the like, on the structure and function of human bodies (same Article, No. 2).

The food composition may comprise a food additive additionally, and unless otherwise specified, the suitability as a "food additive" shall be determined according to the standards and criteria for the corresponding item in accordance with the General Rules and General Test Methods of the Food Additive Code approved by the Ministry of Food and Drug Safety.

The item listed in the "Food Additive Code" may include for example, chemical synthetic products such as ketones, glycine, potassium citrate, nicotinic acid, cinnamic acid, etc., natural additives such as Persimmon color, licorice extract, crystalline cellulose, guar gum, etc., and mixed formulations such as L-glutamine sodium formulations, alkali agents for noodles, preservative formulations, tar color formulations, etc.

The food comprising the active ingredient of the present invention may include confectionery such as bread, rice cakes, dried cakes, candies, chocolates, chewing gum and jam, ice cream produces such as ice cream, ice and ice cream powder, dairy products such as milk, low-fat milk, lactose degradation milk, processed milk, goat milk, fermented milk, butter milk, concentrated milk, milk cream, butter milk, natural cheese, processed cheese, powdered milk and whey, meat products such as processed meat products, processed egg products and hamburgers, fish meat products such as fish cakes, ham, sausage, bacon, etc., noodles such as ramen, dried noodles, fresh noodles, instant fried noodles, gelatinized dried noodles, improved cooked noodles, frozen noodles and pastas, beverages such as fruit juice beverages, vegetable beverages, soybean milk, lactobacillus beverages such as yogurt, etc., and mixed beverages, sauces such as soy sauce, soybean paste, red pepper paste, black soybean paste, mixed paste and vinegar, seasoning food such as tomato ketchup, curry and dressing, margarine, shortening and pizza, but not limited thereto.

In addition thereto, the composition of the present invention may comprise various nutrients, vitamins, electrolytes, flavoring agents, coloring agents, pectic acid and its salts, alginic acid and its salts, organic acids, protective colloid thickeners, pH adjusting agents, stabilizers, preservatives, glycerin, alcohols, carbonating agents used for carbonated beverages, and the like. Moreover, the composition of the present invention may comprise flesh for production of natural fruit juices, fruit juice beverages and vegetable beverages. These ingredients may be used independently or in combination.

The beverage composition comprising the active ingredient of the present invention has no special limitations for other components, and as common beverages, may contain an additional component such as various flavoring agents or natural carbohydrates, or the like. The example of the natural carbohydrates described above common sugars such as monosaccharides (for example, glucose, fructose, etc.); disaccharides (for example, maltose, sucrose, etc.); and polysaccharides (for example, dextrin, cyclodextrin, etc.), and sugar alcohols such as xylitol, sorbitol, erythritol, and the like. As a flavoring agent other than those described above, natural flavoring agents (thaumatin, stevia extracts (for example, rebaudioside A, glycyrrhizin, etc.)) and synthetic flavoring agents (saccharin, aspartame, etc.) are may be advantageously used.

In addition, the composition comprising *Weissella cibaria* WIKIM28 (Accession number KCCM11879P) or its culture as an active ingredient of the present invention may be used as a feed composition or feed additive composition for prevention or improvement of cancer of livestock.

When the composition is produced as a feed additive, the composition may be produced as high concentrate of 20 to 90 % or in a powder or granule form. The feed additive may additionally comprise any one, or one or more of organic acids such as citric acid, fumaric acid, adipic acid, lactic acid, malic acid, etc., or phosphates such as sodium phosphate, potassium phosphate, acidic pyrophosphate, polyphosphate (polyphosphate), etc., or natural anti-oxidants such as polyphenol, catechin, alpha-tocopherol, rosemary extract, vitamin C, green tea extract, licorice extract, keto acid, tannic acid, phytic acid, etc. When produced as feed, the composition may be formulated in a common feed form, and may comprise common feed components together.

The feed and feed additive may further comprise grain, for example, powdered or crushed wheat, oat, barley, corn and rice; plant protein feed, for example, feed having rape, bean and sunflower as a main component; animal protein feed, for example, powdered blood, meat meal, bone dust and fish meal; sugars and dairy products, for example, dried components consisting of various kinds of powdered milk and milk serum powder, and the like, and in addition thereto, may further comprise a nutritional supplement, a digestion and absorption enhancer, a growth promoting agent, and the like.

The feed additive may be administered alone or administered in combination with other feed additive in an edible carrier to animals. In addition, the feed additive may be mixed as top-dressing or directly to animal feed, or easily administered to animals as a separate oral formulation from feed. When the feed additive is administered separately from animal feed, as well-known in the art, it may be produced as an immediate release or sustained release formulation, in combination to a pharmaceutically acceptable edible carrier. This edible carrier may be solid or liquid, for example, corn starch, lactose, sucrose, bean flake, peanut oil, olive oil, sesame oil and propylene glycol. When a solid carrier is used, the feed additive may be a tablet, capsule, powder, troche, lozenge, or non-dispersed form of top-dressing. When a liquid carrier is used, the feed additive may be a formulation of gelatin soft capsule, or syrup or suspension, emulsion or solution.

Furthermore, the feed and feed additive may contain a supplement, for example, a preservative, stabilizer, wetting agent or emulsifier, solution promoter, or the like. The feed additive may be used by adding it to animal feed by salivating, spraying or mixing. The feed or feed additive of the present invention may be applied to many animal feeds including mammals, poultry and fish.

It may be used for pigs, cows, sheep, goats, experimental rodents and pets (e.g.: dogs, cats) in addition to experimental rodents, and the like, as the mammals, and may be used for chickens, turkeys, ducks, geese, pheasants, and quails, and the like, as the poultry, and may be used for trout, and the like as the fish, but not limited thereto.

### Example 1. Culture of strain

### 1-1. Preparation of strains and culture medium

The received *Weissella cibaria* WIKIM28 was inoculated in 1% in 30ml of MRS liquid medium and stationery-cultured at 37°C for 6 hours. After culture, centrifugation was performed at 3500 rpm for 10 minutes to remove the culture medium, and the cells were washed twice with a phosphate-buffered saline (PBS) solution to remove the remaining medium components. Half of the washed cells were placed in a cell culture medium and stationery-cultured for 4 hours at 37°C, 5% CO₂ incubator, and then the culture medium was filtered through a 0.22 µm filter and treated to the cells. Half of the washed cells were diluted in a cell culture medium and treated with live cells.

### 1-2. Preparation of dead cell supernatant

The received *Weissella cibaria* WIKIM28 was inoculated in 1% in 30 ml of MRS liquid medium and stationery-cultured at 37°C for 24 hours. After culture, centrifugation was performed at 3500 rpm for 10 minutes to remove the culture medium, and the cells were washed twice with a phosphate-buffered saline (PBS) solution to remove the remaining medium components. The cells were suspended in PBS solution and heat-treated at 90°C for 30 minutes. After heat treatment, the cells and the supernatant containing the substances in the bacteria eluted from the cells were separated by centrifugation. The supernatant was filtered through a 0.22 µm filter, and then treated to the cells.

### Example 2. In vitro anticancer activity efficacy analysis

### 2-1. Preparation of cell lines

In order to observe the anticancer activity effect of the *Weissella cibaria* WIKIM28, the cell growth rate (cell viability assay) was measured using Cell counting Kit-8. The cells used for the anticancer activity efficacy experiment were non-small cell lung cancer H1650 and A549 cell lines, pancreatic cancer AsPC-1 cell line, bladder cancer T24 cell line, melanoma B16-F10, A375P, and A375SM cell lines, and normal cell line NIH/3T3.

The H1650, A549, AsPC-1, and T24 cell lines and B16-F10, A375P, A375SM, and NIH/3T3 cell lines, respectively, were subcultured under the conditions of 5% CO₂, and 37°C using an RPMI medium and DMEM medium in which 10% FBS (fetal bovine serum) and 1% antibiotics (penicillin/streptomycin) were added, and the cells in the growth period were used for the experiment. The cultured cells were treated with 0.25% trypsin-EDTA in an incubator at 37°C for 3 minutes to detach the cells, and then they were washed with DPBS twice and prepared to be 80% confluency in a 96-well plate.

### 2-2. Cell growth rate (cell viability assay) measurement result

In the present invention, lung cancer (2 types), pancreatic cancer (1 type), bladder cancer (1 type), melanoma (3 types) cells and normal cells (1 type) were cultured and then were treated with *Weissella cibaria* WIKIM28 cells, live cell culture medium, or dead cell supernatant prepared in Example 1 to measure cell growth (cell viability).

For the cell growth rate (cell viability assay), cells were treated with *Weissella cibaria* WIKIM28 cells at a concentration of MOI 50 or 100, live cell culture medium, or dead cell supernatant of 5% (v/v), and then cultured for 24, 48, or 72 hours. After culturing, they were treated with Cell counting Kit-8 reagent, and then the absorbance (OD 450 nm) was measured. Based on the absorbance of cells (control) not treated with *Weissella cibaria* WIKIM28 cells, live cell culture medium, or dead cell supernatant as 100%, the growth rate of cells treated with *Weissella cibaria* WIKIM28 cells, live cell culture medium, or dead cell supernatant was calculated, and the experimental result was described below.

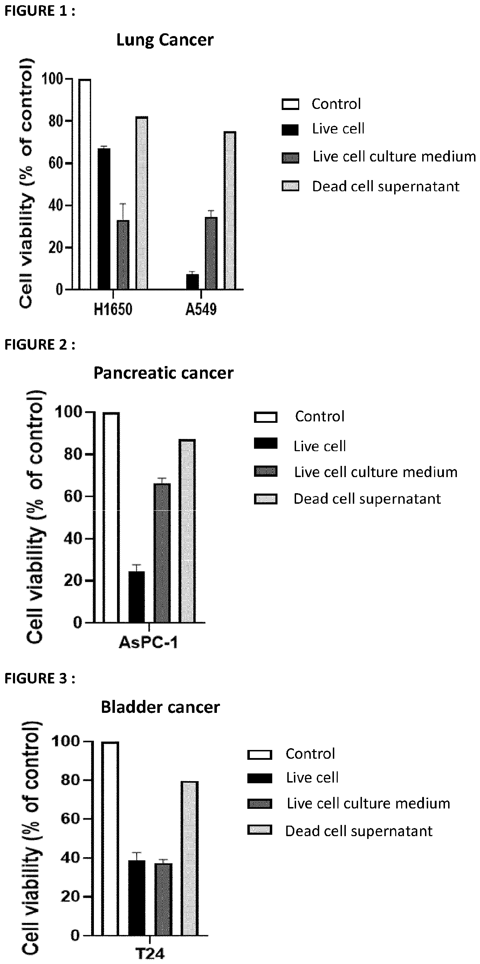

The non-small cell lung cancer H1650 and A549 cell lines were treated with the *Weissella cibaria* WIKIM28 cells, live cell culture medium, or dead cell supernatant prepared in Example 1, and the cell growth rate was measured. The results are shown in FIG. 1.

As shown in FIG. 1, it was confirmed that the cell growth rates of the H1650 cell line after treatment with the *Weissella cibaria* WIKIM28 cells, live culture medium, or supernatant were reduced by 32.9%, 66.8%, and 17.8%, respectively, and the cell growth rates of the A549 cell line after treatment with the *Weissella cibaria* WIKIM28 cells, live culture medium, or supernatant were reduced by 92.5%, 65.3%, and 24.7%, respectively. It can be confirmed that the *Weissella cibaria* WIKIM28 strain has excellent anticancer activation efficacy against lung cancer.

In addition, the pancreatic cancer AsPC-1 cell line was treated with the *Weissella cibaria* WIKIM28 cells, live cell culture medium, or dead cell supernatant prepared in Example 1, and the cell growth rate was measured. The results are shown in FIG. 2.

As shown in FIG. 2, it was confirmed that the cell growth rates of the pancreatic cancer AsPC-1 cell line after treatment with the *Weissella cibaria* WIKIM28 cells, live culture medium, or dead cell supernatant were reduced by 75.7%, 33.7%, and 12.8%, respectively. It can be confirmed that the *Weissella cibaria* WIKIM28 strain has excellent anticancer activation efficacy against pancreatic cancer.

In addition, the bladder cancer T24 cell line was treated with the *Weissella cibaria* WIKIM28 cells, live cell culture medium, or dead cell supernatant, and the cell growth rate was measured. The results are shown in FIG. 3.

As shown in FIG. 3, it was confirmed that the cell growth rates of the bladder cancer T24 cell line after treatment with the *Weissella cibaria* WIKIM28 cells, live culture medium, or dead cell supernatant were reduced by 61.2%, 62.7%, and 20.5%, respectively. It can be confirmed that the *Weissella cibaria* WIKIM28 strain has excellent anticancer activation efficacy against bladder cancer.

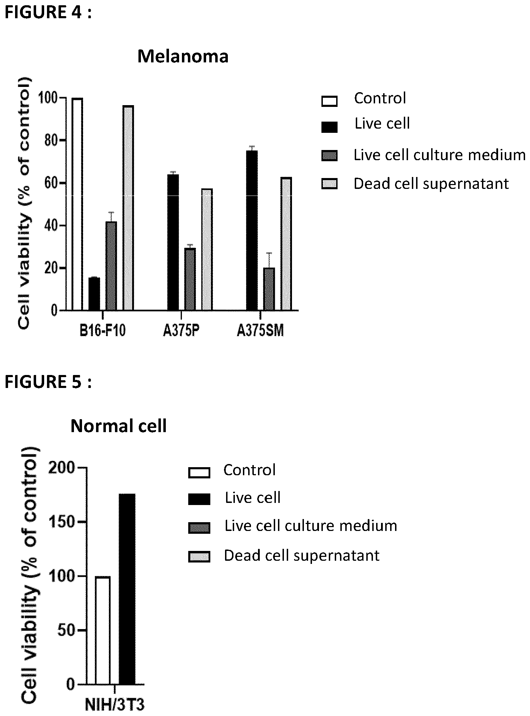

In addition, the melanoma B16-F10, A375P, and A375SM cell lines were treated with the *Weissella cibaria* WIKIM28 cells, live cell culture medium, or dead cell supernatant, and the cell growth rate was measured. The results are shown in FIG. 4.

As shown in FIG. 4, after treatment with the *Weissella cibaria* WIKIM28 cells, live cell culture medium, or dead cell supernatant, the cell growth rate was measured. As a result, it was confirmed that the cell growth rates of the B16-F10 cell line by the cells or live culture medium were reduced by 84.5 and 53.3%, respectively, the cell growth rates of the A375P cell line by the cells, live culture medium, or dead cell supernatant were reduced by 35.8%, 70.4%, and 42.4%, respectively, and the cell growth rates of the A375SM cell line by the cells, live culture medium, or dead cell supernatant were reduced by 24.9%, 79.6%, and 37.2%, respectively.

From the results, it can be confirmed that the *Weissella cibaria* WIKIM28 strain has excellent anticancer activation efficacy against melanoma and skin cancer.

In contrast to the above experiment, normal NIH/3T3 cell lines were treated with the *Weissella cibaria* WIKIM28 cells prepared in Example 1, and the results of measuring the cell growth rate are shown in FIG. 5.

As shown in FIG. 5, it was confirmed that the cell growth rate of the normal NIH/3T3 cell line was increased by 75.9% compared to the control not treated with the *Weissella cibaria* WIKIM28 cells, indicating that the normal cell line showed no toxicity.

### [Accession number]

Name of deposit institution: Korean Culture Center of Microorganisms (Overseas)
Accession number: KCCM11879P
Deposit date: 20151008

## Claims

1. A pharmaceutical composition for preventing or treating cancer, the composition comprising a *Weissella cibaria* or a culture thereof as an active ingredient.

2. The pharmaceutical composition for preventing or treating cancer of claim 1, wherein the cancer is bladder cancer, breast cancer, melanoma, thyroid cancer, parathyroid cancer, rectal cancer, throat cancer, laryngeal cancer, esophageal cancer, pancreatic cancer, stomach cancer, tongue cancer, skin cancer, brain tumor, uterine cancer, double gallbladder cancer, oral cancer, colorectal cancer, perianal cancer, liver cancer, or lung cancer.

3. The pharmaceutical composition for preventing or treating cancer of claim 1, wherein the *Weissella cibaria* has a nucleic acid sequence represented by SEQ ID NO: 1.

4. The pharmaceutical composition for preventing or treating cancer of claim 1, wherein the composition can be administered by a method of oral administration or parenteral administration.

5. The pharmaceutical composition for preventing or treating cancer of claim 4, wherein the method of parenteral administration is intravenous injection, subcutaneous injection, intramuscular injection, intraperitoneal injection, endothelial administration, topical administration, intranasal administration, intrapulmonary administration, or rectal administration.

6. The pharmaceutical composition for preventing or treating cancer of claim 1, wherein the *Weissella cibaria* is in a form of live or dead cell.

7. The pharmaceutical composition for preventing or treating cancer of claim 1, wherein the culture is live cell culture medium or dead cell supernatant.

8. The pharmaceutical composition for preventing or treating cancer of claim 6, wherein the dead cell is stationery-cultured at 25°C to 37°C for 4 hours to 48 hours.

9. A food composition for preventing or alleviating cancer, the composition comprising a *Weissella cibaria* strain or a culture thereof as an active ingredient.

10. The food composition for preventing or alleviating cancer of claim 9, wherein the food is a health functional food.

11. The food composition for preventing or alleviating cancer of claim 9, wherein the food is any one food selected from the group consisting of bread, rice cake, candy, chocolate, gum, ice cream, milk, cheese, processed meat products, processed fish meat products, kimchi, soy sauce, soybean paste, red pepper paste, black soybean paste, cheonggukjang, vinegar, ketchup, curry, dressing, beverage, and fermented milk.

12. A food additive composition for preventing or alleviating cancer, the composition comprising a *Weissella cibaria* strain or a culture thereof as an active ingredient.

13. A feed composition for preventing or alleviating cancer in livestock, the composition comprising a *Weissella cibaria* strain or a culture thereof as an active ingredient.

14. The feed composition for preventing or alleviating cancer in livestock of claim 13, wherein the livestock is any one selected from the group consisting of pig, cow, horse, sheep, rabbit, goat, rat, hamster, guinea pig, dog, cat, chicken, turkey, duck, goose, pheasant, quail, carp, crucian carp, and trout.

15. A feed additive composition for preventing or alleviating cancer in livestock, the composition comprising a *Weissella cibaria* strain or a culture thereof as an active ingredient.
